# EUROPEAN PATENT SPECIFICATION

(11) **EP 1 990 033 B1**
(45) Date of publication and mention of the grant of the patent: **21.04.2010**
(21) Application number: 08251626.1
(22) Date of filing: 07.05.2008
(51) Int. Cl.: A61F 13/15

(54) **Sanitary napkin including body- facing protrusions for preventing side leakage and obliquely arranged embossed channels**
Damenbinde mit körperseitigen Vorsprüngen zur Verhinderung von seitlichem Auslaufen und schräg angeordneten eingeprägten Kanälen
Serviette hygiénique incluant des protubérances côté corps pour empêcher les fuites latérales et des canaux gaufrés obliques

(30) Priority: 08.05.2007 US 745777
(43) Date of publication of application: 12.11.2008
(73) Proprietor: McNeil-PPC, Inc., Skillman, NJ 08558 (US)
(72) Inventor: Marcelo, Ana Maria Elena R., Antipolo City, Rizal Province 1870 (PH); Medina, Maria Socorro F., Cainta, Rizal Province 1900 (PH)
(74) Representative: Kirsch, Susan Edith

(56) References cited:
- EP-A- 1 402 863
- EP-A- 1 493 415
- WO-A-2006/130646
- DE-U1- 29 703 589
- US-A1- 2004 254 554

## Description

### FIELD OF INVENTION

The present invention generally relates to absorbent sanitary napkins and in particular to a sanitary napkin that effectively prevents side leakage while simultaneously providing superior wicking characteristics.

### BACKGROUND OF THE INVENTION

In order for a sanitary napkin to efficiently absorb a large amount of fluid during use it must effectively wick fluid throughout the absorbent structure of the napkin. Absent effective wicking properties menstrual fluid tends to pool in certain regions of the napkin as a result of which the full absorbent capacity of the napkin is not effectively utilized. On the other hand, napkins that exhibit particularly efficient wicking characteristics may exhibit other problems. In particular, such napkins may wick fluid to the longitudinal edge of the absorbent structure resulting in fluid being released from the longitudinal edge of the napkin, i.e. resulting in side leakage. Accordingly, there is a need to provide a sanitary napkin that efficiently wicks fluid yet at the same time effectively prevents side leakage.

EP 1 402 863 discloses a napkin having a preferential bending zone extending obliquely in relation to the longitudinal axis, each preferential bending zone being located solely in an end region and does not extend into the central region of the napkin.

EP 1 493 415 discloses an absorbent article including: a) a body facing surface and b) a garment facing surface wherein at least one embossment is provided on a surface of a material of the absorbent article and the embossment comprises a raised portion having an upper surface that is formed within a depressed area between at least two sidewalls, wherein the surface of the material opposite the embossing pattern is substantially flat.

US 2004/0254554 discloses a fluid-management article, such as a sanitary napkin, comprising a body-faceable, liquid-pervious cover having a top surface, a garment-faceable, liquid-impervious barrier, and an absorbent system intermediate the cover and the barrier. The fluid-management article comprises a plurality of fluid-guiding channels that surround a plurality of isolated protrusions. The channels and the protrusions are formed through the top surface of the cover of the fluid management article. The protrusions have an apex that extends a height greater than about 0.5 millimeters (mm) above at least a portion of the plurality of channels. The protrusions have a number density greater than about 0.15 protrusions/cm².

### SUMMARY OF THE INVENTION

According to a first aspect of the invention, the present invention provides a sanitary napkin including a cover layer, a barrier layer, an absorbent core arranged between the cover layer and the barrier layer, the absorbent core having first and second longitudinally extending edges, a longitudinally extending centerline, a transversely extending centerline, a first and second longitudinal edge, a first and second transverse edge, a first end region and a second end region located longitudinally, a central region arranged between the first and second end regions, a first embossing pattern having a first portion and a second portion, the first portion located in the first end region and the second portion located in the second end region, the first and second portions being spaced from one another in a longitudinal direction of the napkin, each of the first and second portions including a plurality of channels, each one of the channels extending from one side of the longitudinally extending centerline to an opposite side of the longitudinally extending centerline, each one of the channels intersecting at least one other channel at an oblique angle relative thereto and each one of the channels extending across the longitudinally extending centerline at an oblique angle relative thereto, a second embossing pattern arranged in the central region, the second embossing pattern having a first portion located on a first side of the longitudinally extending centerline and a second portion located on a second side of the longitudinally extending centerline, the first and second portions being arranged in spaced relationship from one another in a transverse direction of the napkin, each of the first and second portions of the second embossing pattern being defined by a plurality of interconnected channels, the channels defining a plurality of body facing protrusions, each one of the body facing protrusions being completely surrounded by at least one of the interconnected channels, wherein selected ones of the interconnected channels of the first portion of the second embossing pattern function to define a perimeter of the first portion of the second embossing pattern, wherein the perimeter of the first portion includes an inner arcuate edge, an outer arcuate edge, first and second arcuate transverse edges interconnecting the inner and outer arcuate edges; and selected ones of the interconnected channels of the second portion of the second embossing pattern function to define a perimeter of the second portion of the second embossing pattern, wherein the perimeter of the second portion includes an inner arcuate edge, an outer arcuate edge, and first and second arcuate transverse edges interconnecting the inner and outer arcuate edges; and an embossing-free zone located between the first and second portions of the second embossing pattern in the transverse direction of the napkin.

According to a second aspect of the invention, the present invention provides a sanitary napkin including a cover layer, a barrier layer, an absorbent core arranged between the cover layer and the barrier layer, the absorbent core having a first end second longitudinally extending edges, a longitudinally extending centreline, a transversely extending centreline, a first and second longitudinal edge, a first and second transverse edge, a first end region and a second end region, a central region arranged between the first end second end regions, a first embossing pattern having a first portion and a second portion, the first portion located in the first end region and the second portion located in the second end region, the first and second portions being spaced from one another in a longitudinal direction of the napkin, each of the first and second portions including a plurality of channels, each one of the channels extending from one side of the longitudinally extending centreline to an opposite side of the longitudinally extending centreline, each one of the channels intersecting at least one other channel at an oblique angle relative thereto and each one of the channels extending across the longitudinally extending centreline at an oblique angle relative thereto, each channel being substantially arcuate and having a length of 3.5 cm to 4.5 cm when measured along a path of the channel, each channel having a width from 2 mm to 3.5 mm, and each channel having a depth of 1 to 2 mm when measured from a top surface of the napkin, a second embossing pattern arranged in the central region, the second embossing pattern having a first portion located on a first side of the longitudinally extending centreline and a second portion located on a second side of the longitudinally extending centerline, the first and second portions being arranged in spaced relationship to one another in a transverse direction of the napkin, each of the first and second portions of the second embossing pattern being defined by a plurality of interconnected channels, the channels defining a plurality of body facing protrusions, each one of the body facing protrusions being completely surrounded by at least one of the interconnected channels, selected ones of the interconnected channels of the first portion of the second embossing pattern functioning to define a perimeter of the first portion of the second embossing pattern, selected ones of the interconnected channels of the second portion of the second embossing pattern functioning to define a perimeter of the second portion of the second embossing pattern, the perimeter of the first portion including an inner arcuate edge, an outer arcuate edge, first and second arcuate transverse edges interconnecting the inner and outer arcuate edges, the inner arcuate edge being spaced inwardly relative to the outer arcuate edge, the outer arcuate edge being spaced inwardly relative to the first longitudinally extending edge of the absorbent core by a distance of from 5 mm to 10 mm when measured along the transversely extending centerline of the napkin, the perimeter of the second portion including an inner arcuate edge, an outer arcuate edge, first and second arcuate transverse edges interconnecting the inner and outer arcuate edges, the inner arcuate edge being spaced inwardly relative to the outer arcuate edge, the outer arcuate edge being spaced inwardly relative to the second longitudinally extending edge of the absorbent core by a distance of from 5 mm to 10 mm when measured along the transversely extending centerline of the napkin, the inner arcuate edge of the first portion and the inner arcuate edge of the second portion being spaced from one another by a distance of from 2 cm to 3 cm as measured along the transversely extending centerline, an embossing-free zone located between the inner arcuate edge of the first portion and the inner arcuate edge of the second portion, the embossing-free zone located in an area of the napkin adapted to be arranged over the vaginal opening during use, the embossing-free zone having a width of from 2 cm to 3 cm as measured along the transversely extending centerline and a length of from 5 cm to 10 cm when measured along the longitudinally extending centerline, each one of the protrusions of the second embossing pattern having an apex that is from 1 mm to 2 mm above a bottom surface of a surrounding channel, a surface of the napkin in the embossing-free zone being located a distance of 0.25 mm to 2 mm above each of the apices of each of the protrusions, each one of the protrusions of the second embossing pattern having a surface area in the range of 0.49 cm² to 1.44 cm² and each one of the channels of the second embossing pattern having a width in the range of 0.5 mm to 3 mm, a first end channel located in the first end region and a second end channel located in the second end region, the first and second end channels being spaced from one another in a longitudinal direction of the napkin, each of the first and second end channels being substantially U-shaped, the first end channel being structured and arranged to substantially bound the first portion of the first embossing pattern and the second end channel being structured and arranged to substantially bound the second embossing pattern, each of the first and second end channels having a length of 8 cm to 12 cm as measured along a path of the respective channel, a width of 2 to 4 mm, and a depth of 1 mm to 2 mm as measured from a surface of the napkin.

### BRIEF DESCRIPTION OF THE DRAWINGS

Examples of embodiments of the present invention will now be described with reference to the drawings, in which:
Fig. 1 is a top plan view of a sanitary napkin in accordance with an embodiment of the present invention;
Fig. 2 is a bottom plan view of the sanitary napkin shown in Fig. 1;
Fig. 3 is a exploded perspective view of the napkin shown in Fig. 1 depicting the cover layer, barrier layer and absorbent core thereof;
Fig. 4 is an enlarged detail view of the portion of the napkin encircled by circle 4 in Fig. 1;
Fig. 5 is a cross sectional view taken along the transversely extending centerline 5-5 of the sanitary napkin shown in Fig. 1; and
Fig. 6 is a cross sectional view taken along a portion of longitudinally extending centerline 6-6 of the sanitary napkin shown in Fig. 1.

### DETAILED DESCRITION OF THE INVENTION

The present invention generally relates to disposable absorbent articles such as sanitary napkins, pantiliners, absorbent products for incontinence, and other disposable absorbent articles worn close to a wearer's body. As used herein, the term "sanitary napkin" refers to an article which is worn by females in an undergarment adjacent to the pudendal region and which is intended to absorb and contain various exudates which are discharged from the body (e.g., blood, menses, urine, and the like) and which is intended to be discarded after a single use (i.e., it is not intended to be laundered or otherwise restored or reused). Pantiliners are generally similar to sanitary napkins, except that they typically have lower capacity for absorbing fluids and are generally used to control non-menstrual discharges. Both sanitary napkins and pantiliners are typically attached or secured to a users undergarment and positioned between the undergarment and wearer's pudendal region. Adult incontinence articles, diapers, and interlabial devices are yet other disposable absorbent articles designed to manage various bodily exudates and may benefit from the embodiments of the invention described herein.

Absorbent articles according to the present invention provide superior fluid handling characteristics, and more specifically are particularly adept at preventing side leakage while at the same time providing wicking characteristics required to provide the superior fluid handling characteristics.

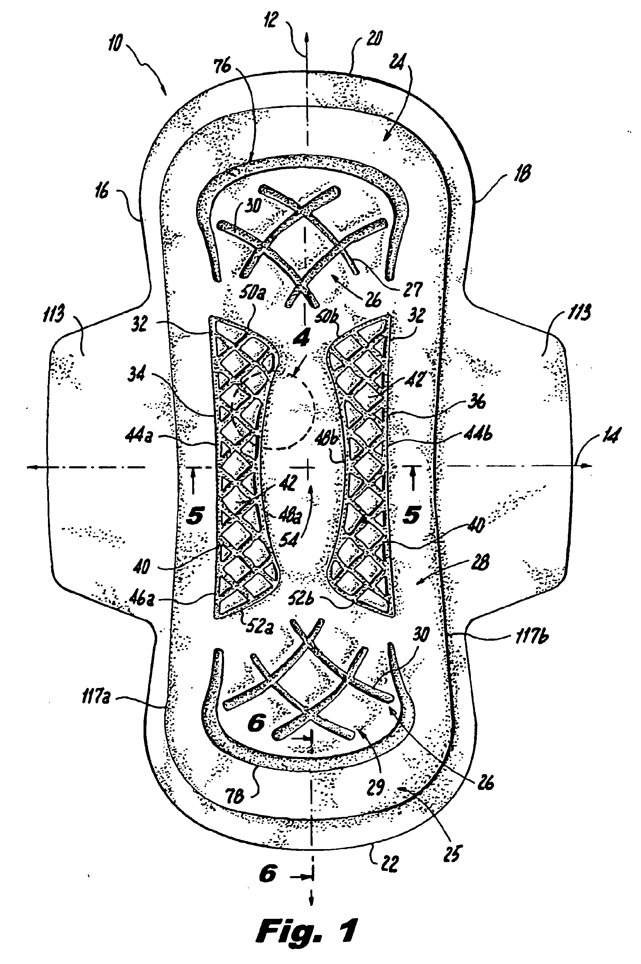

The embodiment of the sanitary napkin 10 according to the present invention is illustrated in Fig. 1. The sanitary napkin 10 includes a first a longitudinally extending centerline 12, a transversely extending centerline 14, a first longitudinal edge 16, a second longitudinal edge 18, a first transverse edge 20, a second transverse edge 22, a first end region 24, a second end region 25, and a central region 28 located between the first end second end regions, 24 and 25.

The napkin 10 further includes a first embossing pattern 26. The first embossing pattern 26 has a first portion 27 arranged in the first end region 24 and a second portion 29 located in the second end region 25, thus the first and second portions 27 and 29 are spaced from one another in the longitudinal direction of the napkin 10. Each of the first portion 27 and second portion 29 comprise a plurality of channels 30, each one of the channels 30 extending from one side of the napkin, across the longitudinally extending centerline 12, to the other side of the napkin. Each one of the channels 30 intersects at least one other channel 30 at an oblique angle relative thereto and each one of the channels 30 extends across the longitudinally extending centerline 12 at an oblique angle relative thereto.

Each of the channels 30 is substantially arcuate in shape and has a length in the range of 2 cm to 6 cm, more preferably from 3.5 cm to 4.5 cm, when measured along the path of the channel 30. Each of the channels 30 has a width in the range of from 1 mm to 10 mm, more preferably from 2 mm to 3.5 mm. Each of the channels 30 has a depth of 0.5 mm to 3 mm, more preferably 1 mm to 2 mm, when measured from a top surface of the napkin 10.

The napkin 10 further includes a second embossing pattern 32. The second embossing pattern 32 is located in the central region 28 of the napkin 10. The second embossing pattern 32 includes a first portion 34 located on a first side of the napkin, i.e. on one side of the longitudinally extending centerline 12, and a second portion 36 located on a second side of the napkin, i.e. on the other side of the longitudinally extending centerline 12.

Each of the first 34 and second portions 36 of the embossing pattern are defined by a plurality of interconnected fluid guiding channels 40, the fluid guiding channels defining therebetween a plurality of body facing protrusions 42. Each of the protrusions 42 are isolated, that is, each of the protrusions 42 is completely surrounded by at least one of the channels 40. The channels 40 that completely surround each protrusion 42 are generally regions of lesser height than the protrusion 42 and may be of higher compression or higher density than the protrusion 42 that is surrounded. The channels 40 of a respective portion, i.e. that is the channels 40 of the first portion 34 or the channels 40 of the second portion 36, are interconnected so as to form a network of continuously interconnected channels 40. The channels 40 also function to define a perimeter 44a of the first portion 34 and a perimeter 44b of the second portion 36.

The perimeter 44a of the first portion 34 includes an outer edge 46a that is substantially parallel to a longitudinally extending peripheral edge 117a of the absorbent core 103 of the napkin 10, and is spaced inwardly relative to the peripheral edge 117a. The perimeter 44a of the first portion 34 further includes an inner arcuate edge 48a that is arranged in inwardly spaced relationship to the outer edge 46a. The outer edge 46a and the inner arcuate edge 48a are interconnected by a first arcuate transverse edge 50a and a second arcuate transverse edge 52a. Preferably the outer edge 46a is spaced inwardly relative to the longitudinally extending peripheral edge 117a of the absorbent core 103 by a distance of 2 mm to 20 mm and more preferably by a distance of 5 mm to 10 mm, when measured along the transversely extending centerline 14 of the napkin 10.

Likewise, the perimeter 44b of the second portion 36 includes an outer edge 46b that is substantially parallel to a longitudinally extending peripheral edge 117b of the absorbent core 103, and spaced inwardly relative to the peripheral edge 117b. The perimeter 44b of the second portion 36 further includes an inner arcuate edge 48b that is arranged in inwardly spaced relationship to the outer edge 46b. The outer edge 46b and the inner arcuate edge 48b are interconnected by a first arcuate transverse edge 50b and a second arcuate transverse edge 52b. Preferably the outer edge 46b is spaced inwardly relative to the longitudinally extending peripheral edge 117b of the absorbent core by a distance of 2 mm to 20 mm and more preferably by a distance of 5 mm to 10 mm, when measured along the transversely extending centerline 14 of the napkin 10.

The first portion 34 and the second portion 36 are spaced from one another in the transverse direction, in particular the inner arcuate edge 48a of the first portion 34 is arranged in spaced relationship to the inner arcuate edge 48b of the second portion 36. The inner arcuate edge 48a and inner arcuate edge 48b function to substantially surround an embossing-free zone 54 zone located therebween. Preferably the embossing-free zone 54 has a width as measured from the inner arcuate edge 48a to the inner arcuate edge 48b along the transversely extending centerline 14 in the range of 1 cm to 5 cm, and more preferably between 2 cm and 3 cm. The embossing-free zone 54 is located in an area of the napkin adapted arranged over the vaginal opening during use. The embossing-free zone 54 preferably has a length of 3 cm to 15 cm when measured along the longitudinally extending centerline 12, and more preferably from 5 cm to 10 cm. The embossing-free zone 54 is considered to end at its top and bottom at the beginning of the channels 30.

A projected area of the protrusions 42 (i.e., the sum of the area of each protrusion 42 within the first and second portions 34 and 36 as projected onto a two dimensional surface, such as is shown in FIG. 1) is greater than a projected area of the channels 40 (i.e., the sum of the area of each channel 40 within the first and second portions 34 and 36 as projected onto a two dimensional surface, such as is shown in FIG. 1). In a preferred embodiment of the invention, a ratio of the projected area of the protrusions 42 to the projected area of the channels 40 is less than 10. In a further preferred embodiment, the ratio is in a range from 3 to 7. Ratios above 10 are less desirable in that the channels 40 occupy too small a portion of the projected area of the sanitary napkin 10 relative to the protrusions 42, thus reducing fluid penetration in the first and second portions 34 and 36.

The channels 40 may be of uniform length or varying length. The plurality of channels 40 and the plurality of protrusions 42 may have various orientations.

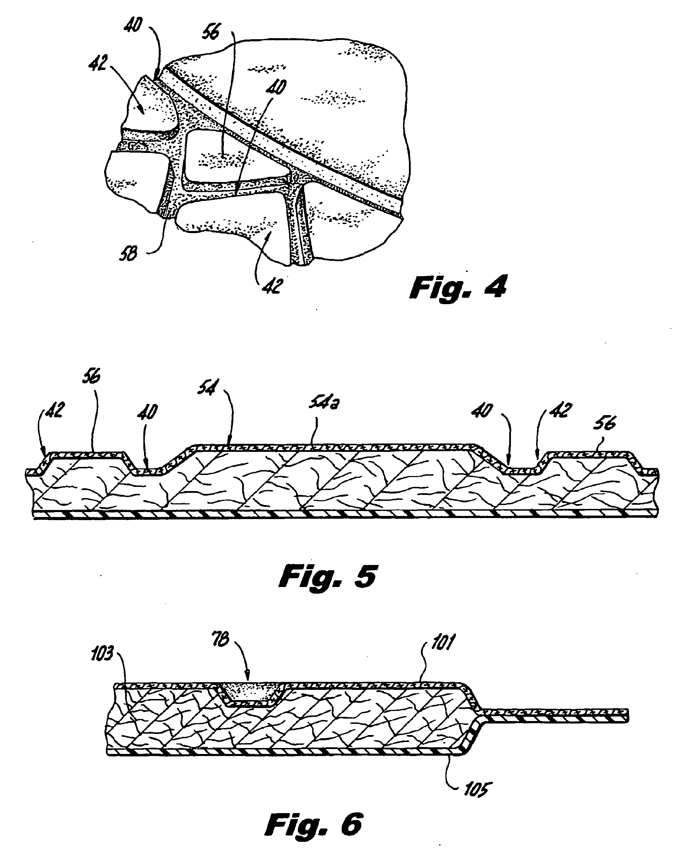

As shown in Fig. 4, each protrusions 42 has an apex 56 that is located 0.5 mm to 3 mm above bottom surface 58 of the surrounding channel 40, more preferably from 1 mm to 2 mm. Stated another way, each channel 40 preferably has a depth relative to an upper surface of the napkin of 0.5 mm to 3 mm, more preferably from 1 mm to 2 mm.

Due to the first and second embossed portions 34 and 36 located on either side of the embossing-free zone 54 the embossing-free zone 54 defines a slightly raised portion relative to apices 56. In particular, as best seen in Fig. 5, the surface of the napkin 54a in the area of the embossing-free zone 54 is preferably 0.25 mm to 2 mm higher than apex 56 more preferably 0.5 mm to 1 mm higher than the apex 56.

The channels 40 are generally shaped such that the bottom surface 58 is flat or concave (i.e., when the sanitary napkin 10 is laid flat, the lower surface 58 is not continuously sloping downward). Thus, the channels 40 are capable of guiding fluid within the first and second portions 34 and 36. Further the channels 40 and protrusions 42 are effective at preventing side leakage of fluid, i.e. leakage caused by movement of fluid in the transverse direction beyond the longitudinal edges of the absorbent system of the napkin.

The sanitary napkin 10 has a caliper that is the thickness of the sanitary napkin 10 as measured in an area outside the embossed areas of the napkin, i.e. outside the first and second embossing patterns 26 and 32. The caliper is measured when the sanitary napkin 10 is in a relaxed, uncompressed state, secured to prevent curling (such as by using 0.907 kg (2 lb). weights arranged on the ends of the napkin to flatten the same), with any release papers removed, and is measured in a region that encompasses all material layers of the sanitary napkin 10. The caliper may be selected based upon desired technical properties desired of the sanitary napkin 10 (e.g. absorbency) or based upon consumer preference. In one embodiment of the invention, the caliper is less than about 5 mm.

In one embodiment of the invention, each protrusion 42 has a surface area in the range of 0.09 cm² to 4 cm² and more preferably in the range of 0.49 cm² to 1.44 cm². Each channel 40 preferably has a width in the range of 0.25 mm to 5 mm and more preferably from 0.5 mm to 3 mm. It has been found that the combination of the channels 40 and protrusions 42, having dimensions in the ranges described above, effectively transmit fluid within the first and second portions 34 and 36 and also are effective at preventing side leakage.

The sanitary napkin 10 may further optionally include a first end channel 76 and a second end channel 78. The first end channel 76 is located in the first end region 24 of the napkin 10 and the second end channel 78 is located in the second end region 26, thus the first end channel 76 is spaced from the second end channel 78 in the longitudinal direction of the napkin. Each of the first and second end channels 76 and 78 are substantially U-shaped and the first 76 and second 78 end channels function to substantially bound the first portion 27 and second portion 29 of the first embossing pattern 26.

"Substantially bound" as used herein means that the outermost point of the first end channel 76 in the longitudinal and transverse directions is located outside the outermost point of any one of the channels 30 located in the first portion 27, and further, none of the channels 30 intersect the first end channel 76. Likewise, the outermost point of the second end channel 78 in the longitudinal and transverse directions is located outside the outermost point of any one of the channels 30 located in the second portion 29, and further, none of the channels 30 intersect the second end channel 76.

Each of the first 76 and second end channels 78 preferably have a length of 5 cm to 26 cm and more preferably from 8 cm to 12 cm, as measured along the path of the channel, and a width of 1 mm to 10 and more preferably 2 mm to 4 mm. Each of the first 76 and second end channels 78 preferably depth of 0.5 mm to 3 mm, more preferably 1 mm to 2 mm as measured form a surface of the napkin.

### Construction of the Sanitary Napkin According to the Invention

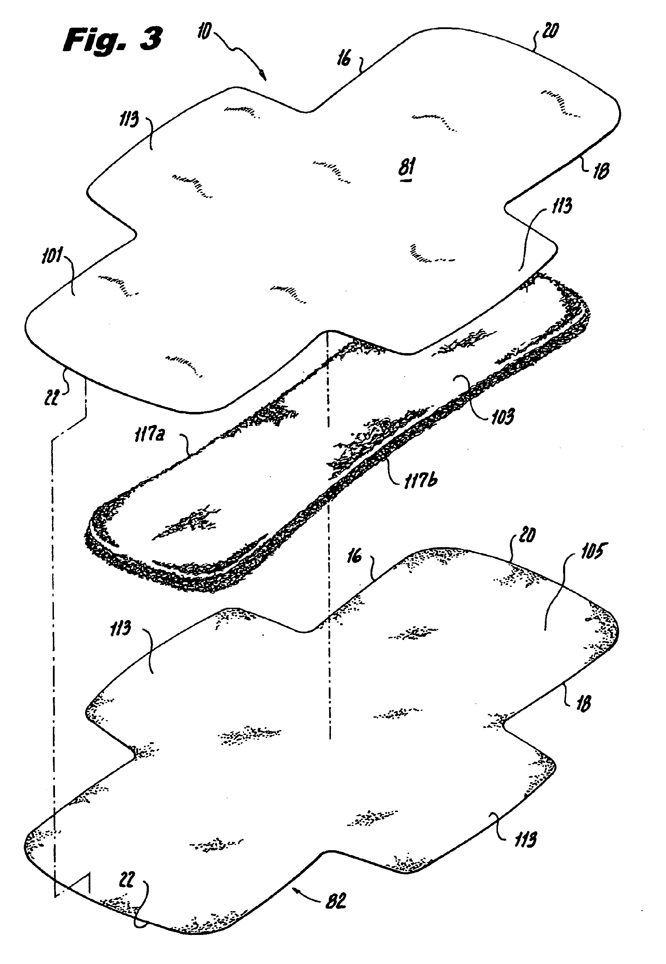

Referring to FIG. 3, the sanitary napkin 10 comprises a fluid-permeable cover layer 101, a liquid-impervious barrier layer 105 and an absorbent core 103 intermediate the fluid-permeable cover layer 101 and the liquid-impervious barrier layer 105. The cover layer 101 has a top surface that forms the body facing surface 81 of the sanitary napkin 10. The cover layer 101 is liquid permeable, and generally compliant, soft feeling, and non-irritating to the user's skin. It can be made from any of the materials conventional for this type of use. The cover layer 101 generally functions to transport fluid away from the wearer into the sanitary napkin 10. In this manner, fluid and moisture are removed from contacting the wearer, thus making the wearer feel dry and comfortable. Non-limiting examples of suitable materials that can be used as the cover layer 101 are woven and nonwoven fabrics formed from polyester, polypropylene, nylon, and/or rayon fibers or the topsheet may be an apertured thermo-plastic film and formed films. The cover layer 101 may optionally be treated with surfactant to manipulate the hydrophobicity/hydrophilicty thereof to facilitate optimal fluid transport properties. The fibers or other materials which make up the cover layer 101 should not collapse or lose their resiliency when subjected to body fluid. The cover layer 101 may be formed from, for example, staple fibers of polypropylene or other suitable materials. The fibers may be oriented by a carding process and thermally bonded via embossing. The basis weight of the cover layer 101 may range from about 10 grams per square meter (gsm) to about 40 gsm.

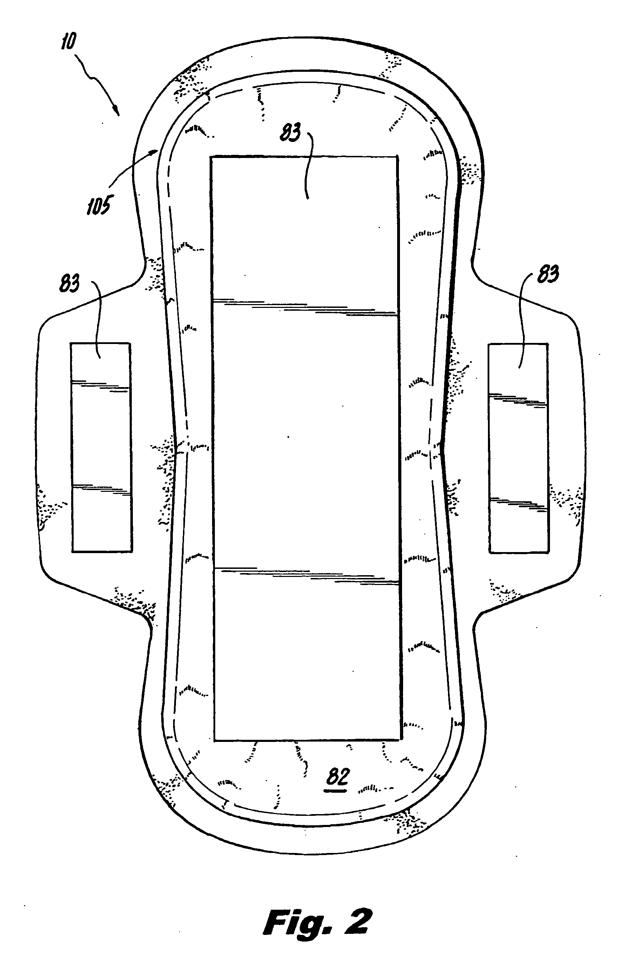

The barrier 105 has a bottom surface that forms the garment facing surface 82 of the napkin 10. The barrier layer 105 is impervious to liquids and, thus, prevents bodily fluid that may be present at the interface between the absorbent core 103 and the barrier layer 105 from soiling the clothing of the user. Suitable materials that may be incorporated into the barrier layer 105 include, for example, embossed or non-embossed polyethylene films, microporous films, and laminated tissue, among other materials. The garment facing surface 82 of the barrier layer 105 is preferably provided with garment attachment adhesive for securing the napkin 10 to an undergarment during use. The garment attachment adhesive is preferably covered with removable release paper 83 to protect the garment attachment adhesive prior to use as seen in Fig. 2.

The absorbent core 103 provides the means for absorbing bodily fluid. Bodily fluid moving inward or "down" from the cover layer 101 is conveyed to the absorbent core 103 which retains the bulk of the fluid until the sanitary napkin 1 is discarded. The absorbent core 103 has a high capacity for absorbing liquids and may be capable of maintaining the definition of the plurality of protrusions 42 and the plurality of channels 40 during the wearing of the sanitary napkin 1.

Examples of material that may be used in the construction of the absorbent core 103 include, for example, cellulosic fibers (preferably wood pulp, but cotton, flax and peat moss are acceptable), synthetic fibers, superabsorbent polymers (SAP) or superabsorbent fibers, as well organic binders and other materials known to the art of manufacturing absorbent core materials. The relative proportion of these materials may be varied to achieve sufficient absorbency, compressibility, and processibility. In one non-limiting example, the absorbent core 103 comprises from about 40 weight percent to about 95 weight percent cellulosic fibers, and from about 5 weight percent to about 60 weight percent superabsorbent polymer.

The absorbent core 103 may include any superabsorbent polymer (SAP). For the purposes of the present invention, the term "superabsorbent polymer" (or "SAP") refers to materials which are capable of absorbing and retaining at least about 10 times their weight in body fluids under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and other material known to the art of absorbent article manufacture.

The sanitary napkin 10 may optionally further include a transfer layer (not shown) and, if present, the transfer layer generally positioned directly underneath the cover layer 101, and the transfer layer directly contacts the absorbent core. The transfer layer provides the means of receiving body fluid from the fluid-pervious cover layer 101 and holding it until the absorbent core has an opportunity to absorb it. The transfer layer is, preferably, more dense than the fluid-pervious cover layer 101 and has a larger proportion of smaller pores than does the latter. These attributes allow the transfer layer to contain body fluid and hold it away from the outer side of the fluid-pervious cover layer 101, thereby preventing the fluid from re-wetting the fluid-pervious cover layer 101 and its surface. However, the transfer layer is preferably not so dense as to prevent the passage of the fluid through the transfer layer and into the underlying absorbent core.

The optional transfer layer may comprise various materials, including, for example, cellulose fibers such as from wood pulp, single component or bicomponent fibers that include thermoplastic materials (such as polyester, polypropylene, polyethylene, among others) in fiber or other forms, rayon, organic binders (such as copolymers of vinyl, acrylic and/or other monomers that may be coated onto thermoplastic fibers or otherwise incorporated into the transfer layer) among other materials known to the art. The transfer layer may, for example, have a basis weight in a range from about 20 grams per square meter (gsm) to about 120 gsm.

While the various material layers (cover, absorbent system, barrier) are described as separate layers, it is within the scope of the invention that one or more of these layers may be formed or integrated together and may actually not be discrete material layers, but rather a unitary layer possessing multiple functional properties.

The channels 30, channels 40 and protrusions 42, and channels 76 and 78, preferably extend into additional material layers of the sanitary napkin 10. For example, in the embodiment of the invention shown in Fig. 1 and Fig. 3, the channels 30, channels 40 and protrusions 42 are formed through the cover layer 101 and the absorbent core 103. By "formed through," the absorbent core 103, it is meant that if one were to follow a top surface absorbent core 103, one would find a plurality of channels 30, channels 40 and protrusions 42 that correspond, register, with or align with the channels 30, channels 40 and protrusions 42 of the cover layer 101. As such, the cover layer 101 follows the contours in the top surface of the absorbent core 103 with no appreciable macroscopic voids present between the two layers.

Referring again to FIG. 3, the absorbent core 103 may be confined to a laterally central region of the sanitary napkin 10 such that the cover layer 101 and barrier 105 extend beyond a peripheral edge 117 of the absorbent core 103. Alternatively, the absorbent core 103 may extend laterally into the flap regions 113. As best seen in Fig. 6, the cover layer 101 and the barrier layer 105 are joined around the entire periphery of the sanitary napkin 10. The cover layer 101 and the barrier layer 105 can be joined by any means commonly used in the art for this purpose such as by gluing, crimping, or heat-sealing. Additional securement of the layers 101, 105 and 103 may be achieved by laminating one or more of these layers together.

The sanitary napkin 10 may be made using various conventional processes known to those of skill in the art, such as, for example, an embossing process in which one or more material layers of the sanitary napkin 10 are subject to mechanical and thermal energy to form channels 30, channels 40 and protrusions 42, and channels 76 and 78.

In accordance with one aspect of the invention, a method of making the sanitary napkin 10 comprises providing a body-faceable, liquid-pervious cover layer 101 having a top surface, a garment-faceable, liquid-impervious barrier layer 105, and an absorbent core 103. The cover layer 101 is arranged on top of the absorbent core 103 and the layers are embossed to provide a plurality of channels 30, channels 40 and protrusions 42, channels 76 and 78 on the surface of the cover layer 101 and the channels 30, channels 40 and protrusions 42, and channels 76 and 78 are formed through the absorbent core 103. The barrier layer 105 is then sealed to the cover layer 101 about a perimeter thereof using heat and pressure.

### EXAMPLES

Inventive Example # 1 - A thin sanitary napkin was constructed including a 22 gsm hot through air bonded cover made from polypropylene/polyethylene bicomponent fibers, a pulp core including 5.5 g of fluff pulp and 0.25 g of superabsorbent, and a 23 gsm polyethylene barrier layer. The sanitary napkin was embossed to include the embossing pattern depicted in Fig. 1 and the sanitary napkin was embossed such that the pattern was formed through the cover and the core but not the barrier.

### Procedure for Measuring Side Leakage

To illustrate the improved fluid handling characteristics of the sanitary napkin of Example #1 described above, and in particular the ability of such a napkin to prevent side leakage, the sanitary napkin of Example #1 was tested according to the test procedure described below. For comparative purposes commercially available napkins were also tested and the results of the above procedures are summarized in Table #1 provided below.

A sanitary napkin according to Example #1 was tested according to the following procedure.
(1) 7.0 ml of a synthetic menstrual fluid was placed in a funnel;
(2) A petri dish was weight and placed underneath the napkin;
(3) The tip of the funnel was arranged at the center of the napkin and was arranged such that the tip of the funnel was spaced 10 mm from the top surface of the napkin;
(4) The valve of the funnel was fully opened;
(5) The pertri dish was observed to determine if there was any leakage from, if any fluid was found in the petri dish the test was considered "failed";
(6) The petri dish was weighed to determine the amount of leakage;
(7) The above described test was repeated for thirty samples;
(8) The percentage of failure was calculated by dividing the number of total failed samples by the total number of samples tested (i.e. 30 samples).

The above described test was conducted on inventive Example #1 and on the following commercially available products, Modess Maxi Cottony Soft Cover, Polyboat, Kotex Soft and Smooth Maxi, Whisper Regular Flow, and Sofy Body Fit Slim.

**TABLE #1**

| Sample | Inventive Example #1 | Modess Maxi Cottony Soft Cover | Polyboat | Kotex Soft and Smooth Maxi | Whisper Regular Flow | Sofy Body Fit Slim |
|---|---|---|---|---|---|---|
| 1 | 0.00 | 2.90 | 4.91 | 4.10 | 0.00 | 0.00 |
| 2 | 0.00 | 4.16 | 4.03 | 4.02 | 1.23 | 0.00 |
| 3 | 0.00 | 2.03 | 4.27 | 4.33 | 0.00 | 3.64 |
| 4 | 0.00 | 4.09 | 4.77 | 1.04 | 0.00 | 2.75 |
| 5 | 0.00 | 3.55 | 5.32 | 2.94 | 0.00 | 0.00 |
| 6 | 3.02 | 1.46 | 3.16 | 4.53 | 3.54 | 0.00 |
| 7 | 0.00 | 0.00 | 6.15 | 4.06 | 2.85 | 2.29 |
| 8 | 0.00 | 0.00 | 3.69 | 1.38 | 0.00 | 2.80 |
| 9 | 0.00 | 4.27 | 5.79 | 1.47 | 0.00 | 0.00 |
| 10 | 0.00 | 2.32 | 4.48 | 2.94 | 0.00 | 0.75 |
| 11 | 0.00 | 0.00 | 4.62 | 3.95 | 0.00 | 0.00 |
| 12 | 0.00 | 0.00 | 5.38 | 1.49 | 0.00 | 2.57 |
| 13 | 0.00 | 3.63 | 5.21 | 0.30 | 3.79 | 1.00 |
| 14 | 0.00 | 4.34 | 4.02 | 2.67 | 0.00 | 4.01 |
| 15 | 0.00 | 0.25 | 3.70 | 2.29 | 0.00 | 0.00 |
| 16 | 0.00 | 4.36 | 4.91 | 4.84 | 0.00 | 0.00 |
| 17 | 1.93 | 0.00 | 2.75 | 4.39 | 0.71 | 0.00 |
| 18 | 0.00 | 0.00 | 5.03 | 4.15 | 0.00 | 0.00 |
| 19 | 0.00 | 0.00 | 5.69 | 4.41 | 2.06 | 2.95 |
| 20 | 0.00 | 0.00 | 4.04 | 1.94 | 0.00 | 2.92 |
| 21 | 0.00 | 0.56 | 5.17 | 4.04 | 2.06 | 2.99 |
| 22 | 0.00 | 0.85 | 4.96 | 5.45 | 0.00 | 4.50 |
| 23 | 0.00 | 1.79 | 4.15 | 4.60 | 2.44 | 0.00 |
| 24 | 0.00 | 1.48 | 5.35 | 4.14 | 2.73 | 0.00 |
| 25 | 0.00 | 0.00 | 4.91 | 4.34 | 2.70 | 2.57 |
| 26 | 0.00 | 0.00 | 4.66 | 5.06 | 0.00 | 0.00 |
| 27 | 0.00 | 1.28 | 3.91 | 3.70 | 0.00 | 4.21 |
| 28 | 0.00 | 0.00 | 5.58 | 5.20 | 0.00 | 1.84 |
| 29 | 0.00 | 0.00 | 5.20 | 2.98 | 0.00 | 0.44 |
| 30 | 0.00 | 0.36 | 5.07 | 3.65 | 3.88 | 0.00 |
| Total Number | | | | | | |
| of Samples | 30 | 30 | 30 | 30 | 30 | 30 |
| Leakage | | | | | | |
| Occurrence | 2 | 18 | 30 | 30 | 11 | 16 |
| Percentage of | | | | | | |
| leakage | | | | | | |
| occurrence | 6.67 | 60.00 | 100.00 | 100.00 | 36.67 | 53.33 |

As illustrated by the above test the napkin according to the present invention exhibited superior fluid handling characteristics and in particular significantly reduced the occurrence of side leakage.

## Claims

1. A sanitary napkin (10) comprising:
a cover layer (101);
a barrier layer (105);
an absorbent core (103) arranged between the cover layer (101) and the barrier layer (105), the absorbent core (103) having first and second longitudinally extending edges (117a and 117b);
a longitudinally extending centreline (12);
a transversely extending centreline (14);
a first and second longitudinal edge (16 and 18);
a first and second transverse edge (20 and 22);
a first end region (24) and a second end region (25) located longitudinally,
a central region (28) arranged between the first and second end regions (24 and 25);
a first embossing pattern (26) having a first portion (27) and a second portion (29), the first portion (27) located in the first end region (24) and the second portion (29) located in the second end region (25), the first and second portions (27, 29) being spaced from one another in a longitudinal direction of the napkin (10), each of the first and second portions (27 and 29) comprising a plurality of channels (30), each one of the channels (30) extending from one side of the longitudinally extending centerline to an opposite side of the longitudinally extending centerline, each one of the channels (30) intersecting at least another channel (30) at an oblique angle relative thereto and each one of the channels (30), extending across the longitudinally extending centreline (12) at an oblique angle relative thereto;
a second embossing pattern (32) arranged in the central region (28), the second embossing pattern (32) having a first portion (34) located on a first side of the longitudinally extending centerline and a second portion (36) located on a second side of the longitudinally extending centerline, the first and second portions (34 and 36) being arranged in spaced relationship from one another in a transverse direction of the napkin (10), each of the first and second portions (34, 36) of the second embossing pattern (32) being defined by a plurality of interconnected channels (40), the channels (40) defining a plurality of body facing protrusions (42);
each one of the body facing protrusions (42) being completely surrounded by at least one of the interconnected channels (40);
wherein selected ones of the interconnected channels (40) of the first portion (34) of the second embossing pattern (32) function to define a perimeter (44a) of the first portion (34) of the second embossing pattern (32), and selected ones of the interconnected channels (40) of the second portion (36) of the second embossing pattern (32) function to define a perimeter (44b) of the second portion (36) of the second embossing pattern (32);
wherein the perimeter (44a) of the first portion (34) includes an inner arcuate edge (48a), an outer arcuate edge (46a), first and second arcuate transverse edges (50a and 52a) interconnecting the inner and outer arcuate edges (48a and 46a);
wherein the perimeter (44b) of the second portion (36) includes an inner arcuate edge (48b), an outer arcuate edge (46b), and first and second arcuate transverse edges (50b and 52b) interconnecting the inner and outer arcuate edges (48b and 46b); and
an embossing-free zone (54) located between first and second portions (34 and 36) of the second embossing pattern (32) in the transverse direction of the napkin.

2. The sanitary napkin (10) according to claim 1, wherein each channel (30) of the first embossing pattern (26) is substantially arcuate.

3. The sanitary napkin (10) according to claim 1 or claim 2, wherein each channel (30) of the first embossing pattern (26) has a length of 2.0 cm to 6.0 cm, when measured along a path of the channel.

4. The sanitary napkin (10) according to claim 3 wherein each channel (30) of the first embossing pattern (26) has a length of 3.5 cm to 4.5 cm when measured along a path of the channel.

5. The sanitary napkin (10) according to any preceding claim, wherein each channel (30) of the first embossing pattern (26) has a width from 2 mm to 3.5 mm.

6. The sanitary napkin (10) according any preceding claim, wherein each channel (30) of the first embossing pattern (26) has a depth of 1 to 2 mm when measured from a top surface of the napkin.

7. The sanitary napkin (10) according to claim 1, wherein the inner arcuate edge (48a) is spaced inwardly relative to the outer arcuate edge (46a) and the outer arcuate edge (46a) is spaced inwardly relative to the first longitudinally extending edge (117a) of the absorbent core (103).

8. The sanitary napkin (10) according to claim 7, wherein the outer arcuate edge (46a) is spaced inwardly relative to the first longitudinally extending edge (117a) of the absorbent core (103) by a distance of from 5 mm to 10 mm when measured along the transversely extending centreline (14) of the napkin (10).

9. The sanitary napkin according to claim 1, wherein the inner arcuate edge (48b) is spaced inwardly relative to the outer arcuate edge (46b) and the outer arcuate edge (46b) is spaced inwardly relative to the second longitudinally extending edge (117b) of the absorbent core (103).

10. The sanitary napkin (10) according to claim 9, wherein outer arcuate edge (46b) is spaced inwardly relative to the second longitudinally extending edge (117b) of the absorbent core (103) by a distance of from 5 mm to 10 mm when measured along the transversely extending centerline (14) of the napkin (10).

11. The sanitary napkin (10) according to any one of claims 6 to 10, wherein an inner arcuate edge (48a) of the first portion (34) and an inner arcuate edge (48b) of the second portion (36) are spaced from one another by a distance of from 2 cm to 3 cm as measured along the transversely extending centreline (14).

12. The sanitary napkin (10) according to any preceding claim, wherein the embossing-free zone (54) located in an area of the napkin (10) adapted to be arranged over the vaginal opening during use.

13. The sanitary napkin (10) according to any preceding claim, wherein the embossing-free zone (54) has a width of from 2 cm to 3 cm as measured along the transversely extending centreline (14).

14. The sanitary napkin (10) according to any preceding claim, wherein the embossing-free zone (54) has a length of from 5 cm to 1 cm when measured along the longitudinally extending centreline (12).

15. The sanitary napkin (10) according to any preceding claim, further comprising a first end channel (76) located in the first end region (24) and a second end channel (78) located in the second end region (25).

16. The sanitary napkin (10) according to claim 15, wherein the first and second end channels (76 and 78) are spaced from one another in a longitudinal direction of the napkin (10), the first end channel (76) being structured and arranged to substantially bound the first portion (27) of the first embossing pattern (26) and the second end channel (78) being structured and arranged to substantially bound the second portion (29) of the first embossing pattern (26).

17. A sanitary napkin (10) according to each of claims 1 to 11, wherein
the embossing-free zone (54) located between the inner arcuate edge (48a) of the first portion (34) and the inner arcuate edge (48b) of the second portion (36), the embossing-free zone (54) located in an area of the napkin (10) adapted to be arranged over the vaginal opening during use, the embossing-free zone (54) having a width of from 2 cm to 3 cm as measured along the transversely extending centreline (14) and a length of from 5 cm to 10 cm when measured along the longitudinally extending centreline (12);
each one of the protrusions (42) of the second embossing pattern (32) having an apex (56) that is from 1 mm to 2 mm above a bottom surface (58) of a surrounding channel (40);
a surface of the napkin (10) in the embossing-free zone (54) being located a distance of 0.25 mm to 2 mm above each of the apices (56) of each of the protrusions (42);
each one of the protrusions (42) of the second embossing pattern (32) having a surface area in the range of 0.49 cm² to 1.44 cm² and each one of the channels (40) of the second embossing pattern (32) having a width in the range of 0.5 mm to 3 mm;
a first end channel (76) located in the first end region (24) and a second end channel (78) located in the second end region (25), the first and second end channels (76 and 78) being spaced from one another in a longitudinal direction of the napkin (10), each of the first and second end channels (76 and 78) being substantially U-shaped, the first end channel (76) being structured and arranged to substantially bound the first portion (27) of the first embossing pattern (26) and the second end channel (78) being structured and arranged to substantially bound the second portion (29) of the first embossing pattern (26), each of the first and second end channels (76 and 78) having a length of 8 cm to 12 cm as measured along a path of the respective channel, a width of 2 to 4 mm, and a depth of 1 mm to 2 mm as measured from a surface of the napkin (10).

## Patentansprüche

1. Damenbinde (10), die aufweist:
eine Deckschicht (101);
eine Barrierenschicht (105);
einen absorbierenden Kern (103), der zwischen der Deckschicht (101) und der Barrierenschicht (105) angeordnet ist, wobei der absorbierende Kern (103) eine erste und eine zweite sich in Längsrichtung erstreckende Kante (117a und 117b) hat;
eine sich in Längsrichtung erstreckende Mittellinie (12);
eine sich in Querrichtung erstreckende Mittellinie (14);
eine erste und eine zweite längsverlaufende Kante (16 und 18);
eine erste und eine zweite querverlaufende Kante (20 und 22);
einen ersten Endbereich (24) und einen zweiten Endbereich (25), die in Längsrichtung angeordnet sind;
einen Mittelbereich (28), der zwischen dem ersten und dem zweiten Endbereich (24 und 25) angeordnet ist;
ein erstes Prägemuster (26) mit einem ersten Teilbereich (27) und einem zweiten Teilbereich (29), wobei sich der erste Teilbereich (27) in dem ersten Endbereich (24) befindet und sich der zweite Teilbereich (29) in dem zweiten Endbereich (25) befindet, wobei der erste und der zweite Teilbereich (27, 29) voneinander in einer Längsrichtung der Binde (10) beabstandet sind, wobei sowohl der erste als auch der zweite Teilbereich (27 und 29) eine Vielzahl von Kanälen (30) aufweist, wobei jeder der Kanäle (30) von einer Seite der sich in Längsrichtung erstreckenden Mittellinie zu einer gegenüberliegenden Seiten der sich in Längsrichtung erstreckenden Mittellinie verläuft, wobei jeder der Kanäle (30) wenigstens einen weiteren Kanal (30) unter einem schrägen Winkel in Bezug auf diesen schneidet und jeder der Kanäle (30) sich über die sich in Längsrichtung erstreckende Mittellinie (12) unter einem schrägen Winkel in Bezug auf diese erstreckt;
ein zweites Prägemuster (32), das in dem Mittelbereich (28) angeordnet ist, wobei das zweite Prägemuster (32) einen ersten Teilbereich (34), der sich auf der ersten Seite der sich in Längsrichtung erstreckenden Mittellinie befindet, und einen zweiten Teilbereich (36), der sich auf einer zweiten Seite der sich in Längsrichtung erstreckenden Mittellinie befindet, hat, wobei der erste und der zweite Teilbereich (34 und 36) in einer Lagebeziehung zueinander in einer Querrichtung der Binde (10) beabstandet angeordnet sind, wobei sowohl der erste als auch der zweite Teilbereich (34, 36) des zweiten Prägemusters (32) durch eine Vielzahl miteinander verbundener Kanäle (40) definiert ist, wobei die Kanäle (40) eine Vielzahl von körperseitigen Vorsprüngen (42) definieren;
wobei jeder der körperseitigen Vorsprünge (42) vollständig von wenigstens einem der miteinander verbundenen Kanäle (40) umgeben ist;
wobei ausgewählte der miteinander verbundenen Kanäle (40) des ersten Teilbereichs (34) des zweiten Prägemusters (32) so wirken, dass sie einen Umfangsbereich (44a) des ersten Teilbereiches (34) des zweiten Prägemusters (32) definieren, und ausgewählte der miteinander verbundenen Kanäle (40) des zweiten Teilbereiches (36) des zweiten Prägemusters (32) so wirken, dass sie einen Umfangsbereich (44b) des zweiten Teilbereiches (36) des zweiten Prägemusters (32) definieren;
wobei der Umfangsbereich (44a) des ersten Teilbereiches (34) eine innere gebogene Kante (48a), eine äußere gebogene Kante (46a), eine erste und eine zweite gebogene, querverlaufende Kante (50a und 52a), die die innere und die äußere gebogene Kante (18a und 16a) verbinden, umfasst;
wobei der Umfangsbereich (44b) des zweiten Teilbereiches (36) eine innere gebogene Kante (48b), eine äußere gebogene Kante (46b) und eine erste und eine zweite gebogene querverlaufende Kante (50b und 52b), die die innere und die äußere gebogene Kante (48b und 46b) verbinden, umfasst; und
eine prägungsfreie Zone (54), die sich zwischen dem ersten und dem zweiten Teilbereiche (34 und 36) des zweiten Prägemusters (32) in der Querrichtung der Binde befindet.

2. Damenbinde (10) nach Anspruch 1, bei der jeder Kanal (30) des ersten Prägemusters (26) im Wesentlichen bogenförmig ist.

3. Damenbinde (10) nach Anspruch 1 oder Anspruch 2, bei der jeder Kanal (30) des ersten Prägemusters (26) eine Länge von 2.0 cm bis 6.0 cm, wenn entlang einem Weg des Kanals gemessen wird, hat.

4. Damenbinde (10) nach Anspruch 1, bei der jeder Kanal (30) des ersten Prägemusters (26) eine Länge von 3.5 cm bis 4.5 cm, wenn entlang einem Weg des Kanals gemessen wird, hat.

5. Damenbinde (10) nach einem der vorangehenden Ansprüche, bei der jeder Kanal (30) des ersten Prägemusters (26) eine Breite von 2 mm bis 3.5 mm hat.

6. Damenbinde (10) nach einem der vorangehenden Ansprüche, bei der jeder Kanal (30) des ersten Prägemusters (26) eine Tiefe von 1 bis 2 mm, wenn von einer oberen Fläche der Binde her gemessen wird, hat.

7. Damenbinde (10) nach Anspruch 1, bei der die innere gebogene Kante (48a) in Bezug auf die äußere gebogene Kante (46a) nach innen beabstandet ist und die äußere gebogene Kante (46a) in Bezug auf die erste, sich in Längsrichtung erstreckende Kante (117a) des absorbierenden Kerns (103) nach innen beabstandet ist.

8. Damenbinde (10) nach Anspruch 7, bei der die äußere gebogene Kante (46a) in Bezug auf die erste, sich in Längsrichtung erstreckende Kante (117a) des absorbierenden Kerns (103) in einem Abstand von 5 mm bis 10 mm, wenn entlang der sich in Querrichtung erstreckenden Mittellinie (14) der Binde (10) gemessen wird, nach innen beabstandet ist.

9. Damenbinde nach Anspruch 1, bei der die innere gebogene Kante (48b) in Bezug auf die äußere gebogene Kante (46b) nach innen beabstandet ist und die äußere gebogene Kante (46b) in Bezug auf die zweite, sich in Längsrichtung erstreckende Kante (117b) des absorbierenden Kerns (103) nach innen beabstandet ist.

10. Damenbinde (10) nach Anspruch 9, bei der die äußere gebogene Kante (46b) in Bezug auf die zweite, sich in Längsrichtung erstreckende Kante (117b) des absorbierenden Kerns (103) in einem Abstand von 5 mm bis 10 mm, wenn entlang der sich in Querrichtung erstreckenden Mittellinie (14) der Binde (10) gemessen wird, nach innen beabstandet ist.

11. Damenbinde (10) nach einem der Ansprüche 6 bis 10, bei der eine innere bogenförmige Kante (48a) des ersten Teilbereichs (34) und eine innere bogenförmige Kante (48b) des zweiten Teilbereiches (36) voneinander mit einer Entfernung von 2 cm bis 3 cm, wenn entlang der sich in Querrichtung erstreckenden Mittelinie (14) gemessen wird, beabstandet sind.

12. Damenbinde (10) nach einem der vorangehenden Ansprüche, bei der sich die prägungsfreie Zone (54) in einem Gebiet der Binde (10) befindet, das dazu ausgelegt ist, während der Verwendung über der Vaginaöffnung angeordnet zu werden.

13. Damenbinde (10) nach einem der vorangehenden Ansprüche, bei der die prägungsfreie Zone (54) eine Breite von 2 cm bis 3 cm, wenn entlang der sich in Querrichtung erstreckenden Mittelinie (14) gemessen wird, hat.

14. Damenbinde (10) nach einem der vorangehenden Ansprüche, bei der die prägungsfreie Zone (54) eine Länge von 5 cm bis 10 cm, wenn entlang der sich in Längsrichtung erstreckenden Mittellinie (12) gemessen wird, hat.

15. Damenbinde (10) nach einem der vorangehenden Ansprüche, die weiter einen ersten Endkanal (76), der sich in dem ersten Endbereich (24) befindet, und einen zweiten Endkanal (78), der sich in dem zweiten Endbereich (25) befindet, aufweist.

16. Damenbinde (10) nach Anspruch 15, bei der der erste und der zweiten Endkanal (76 und 78) voneinander in einer Längsrichtung der Binde (10) beabstandet sind, wobei der erste Endkanal (16) so strukturiert und angeordnet ist, dass er im Wesentlichen den ersten Teilbereich (27) des ersten Prägemusters (26) begrenzt, und der zweite Endkanal (78) so strukturiert und angeordnet ist, dass er im Wesentlichen den zweiten Teilbereich (29) des ersten Prägemusters (26) begrenzt.

17. Damenbinde (10) nach jedem der Ansprüche 1 bis 11, bei der
die prägungsfreie Zone (54) sich zwischen der inneren bogenförmigen Kante (48a) des ersten Teilbereiches (34) und der zweiten gebogenen Kante (48b) des zweiten Teilbereiches (36) befindet, wobei sich die prägungsfreie Zone (54) in einem Gebiet der Binde (10) befindet, dass dazu ausgelegt ist, während der Verwendung über der Vaginaöffnung angeordnet zu werden, wobei die prägungsfreie Zone (54) eine Breite von 2 cm bis 3 cm, wenn entlang der sich in Querrichtung erstreckenden Mittelinie (14) gemessen wird und eine Länge von 5 cm bis 10 cm, wenn entlang der sich in Längsrichtung erstreckenden Mittelinie (12) gemessen wird, hat;
wobei jeder der Vorsprünge (42) des zweiten Prägemusters (32) einen Scheitel (56) hat, der 1 mm bis 2 mm oberhalb einer unteren Fläche (58) eines umgebenden Kanals (10) liegt;
wobei sich eine Fläche der Binde (10) in der prägungsfreien Zone (54) in einem Abstand von 0.25 mm bis 2 mm oberhalb jedes der Scheitel (56) jedes der Vorsprünge (42) befindet;
wobei jeder der Vorsprünge (42) des zweiten Prägemusters (32) ein Oberflächengebiet in dem Bereich von 0.49 cm² bis 1.44 cm² hat und jeder der Kanäle (40) des zweiten Prägemusters (32) eine Breite in dem Bereich von 0.5 mm bis 3 mm hat;
wobei ein erster Endkanal (76) sich in dem ersten Endbereich (24) befindet und ein zweiter Endkanal (78) sich in dem zweiten Endbereich (25) befindet, wobei der erste und der zweite Endkanal (76 und 78) voneinander in einer Längsrichtung der Binde (10) beabstandet sind, wobei der erste und der zweite Endkanal (76 und 78) im Wesentlichen U-förmig sind, wobei der erste Endkanal (76) so strukturiert und angeordnet ist, dass er im Wesentlichen den ersten Teilbereich (27) des ersten Prägemusters (26) begrenzt, und der zweite Endkanal (78) so strukturiert und angeordnet ist, dass er im Wesentlichen den zweiten Teilbereich (29) des ersten Prägemusters (26) begrenzt, wobei sowohl der erste als auch der zweite Endkanal (76 und 78) eine Länge von 8 cm bis 12 cm, wenn entlang einem Weg des jeweiligen Kanals gemessen wird, eine Bereite von 2 bis 4 mm und eine Tiefe von 1 mm bis 2 mm, wenn von einer Fläche der Binde (10) her gemessen wird, hat.

## Revendications

1. Serviette hygiénique (10) comprenant :
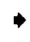 une couche de couverture (101) ;
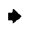 une couche barrière (105) ;
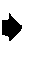 un noyau absorbant (103) disposé entre la couche de couverture (101) et la couche barrière (105), le noyau absorbant (103) ayant un premier et un second bords s'étendant longitudinalement (117a et 117b) ;
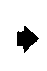 une ligne centrale s'étendant longitudinalement (12) ;
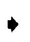 une ligne centrale s'étendant transversalement (14) ;
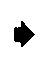 un premier et un second bords longitudinaux (16 et 18) ;
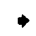 un premier et un second bords transversaux (20 et 22) ;
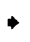 une première région d'extrémité (24) et une seconde région d'extrémité (25) situées longitudinalement,
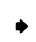 une zone centrale (28) disposée entre la première et la seconde zones d'extrémité (24 et 25) ;
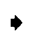 un première motif gaufré (26) ayant une première partie (27) et une seconde partie (29), la première partie (27) étant située dans la première zone d'extrémité (24) et la seconde partie (29) étant située dans la seconde zone d'extrémité (25), la première et la seconde parties (27, 29), étant espacées l'une de l'autre dans une direction longitudinale de la serviette (10), chacune de la première et de la seconde parties (27 et 29) comprenant une pluralité de canaux (30), chacun des canaux (30) s'étendant d'un côté de la ligne centrale s'étendant longitudinalement vers un côté opposé de la ligne centrale s'étendant longitudinalement, chacun des canaux (30) coupant au moins un autre canal (30) selon un angle oblique relativement à celui-ci et chacun des canaux (30) s'étendant à travers la ligne centrale s'étendant longitudinalement (12) selon un angle oblique relativement à celui-ci ;
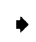 un second motif gaufré (32) disposé dans la zone centrale (28), le second motif gaufré (32) ayant une première partie (34) située sur le premier côté de la ligne centrale s'étendant longitudinalement et une seconde partie (36) située sur un second côté de la ligne centrale s'étendant longitudinalement, la première et la seconde parties (34 et 36), étant disposées dans une relation espacée l'une de l'autre, dans une direction transversale de la serviette (10), chacune des première et seconde parties (34, 36) du second motif gaufré (32) étant définie par une pluralité de canaux interconnectés (40), les canaux (40) définissant une pluralité de protubérances orientées vers le corps (42) ;
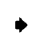 chacune des protubérances faisant face au corps (42) étant totalement entourée d'au moins un des canaux interconnectés (40) ;
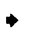 dans lequel les canaux interconnectés choisis (40) de la première partie (34) du second motif gaufré (32) fonctionnent pour définir un périmètre (44a) de la première partie (34) du second motif gaufré (32), et les canaux interconnectés choisis (40) de la seconde partie (36) du second motif gaufré (32) fonctionnent pour définir un périmètre (11b) de la seconde partie (36) du second motif gaufré (32) ;
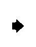 dans lequel le périmètre (44a) de la première partie (34) comprend un bord arqué interne (48a), un bord arqué externe (46a), un premier et un second bords transversaux arqués (40a et 52a), interconnectant les bords arqués interne et externe (18a et 16a) ;
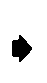 dans lequel le périmètre (11b) de la seconde partie (36) comprend un bord arqué interne (48b), un bord arqué externe (16b) et le premier et le second bords transversaux (50b et 52b) interconnectant les bords arqués interne et externe (48b et 46b) ; et
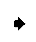 une zone dénuée de gaufrage (54) située entre la première et la seconde parties (34 et 36) du second motif gaufré (32) dans le sens transversal de la serviette.

2. Serviette hygiénique (10) selon la revendication 1, dans laquelle chaque canal (30) du premier motif gaufré (26) est sensiblement arqué.

3. Serviette hygiénique (10) selon la revendication 1 ou la revendication 2, dans laquelle chaque canal (30) du premier motif gaufré (26) a une longueur de 2,0 cm à 6,0 cm, quand elle est mesurée le long d'un chemin du canal.

4. Serviette hygiénique (10) selon la revendication 3, dans laquelle chaque canal (30) du premier motif gaufré (26) a une longueur de 3,5 cm à 4,5 cm quand elle est mesurée le long d'un chemin du canal.

5. Serviette hygiénique (10) selon l'une quelconque des revendications précédentes, dans laquelle chaque canal (30) du premier motif gaufré (26) a une largeur de 2 mm à 3,5 mm.

6. Serviette hygiénique (10) selon l'une quelconque des revendications précédentes, dans laquelle chaque canal (30) du premier motif gaufré (26) a une profondeur de 1 à 2 mm, quand elle est mesurée depuis une surface supérieure de la serviette.

7. Serviette hygiénique (10) selon la revendication 1, dans laquelle le bord arqué interne (18a) est espacé vers l'intérieur relativement au bord arqué extérieur (46a) et le bord arqué extérieur (46a) est espacé vers l'intérieur relativement au premier bord s'étendant longitudinalement (117a) du noyau absorbant (103).

8. Serviette hygiénique (10) selon la revendication 7, dans laquelle le bord arqué extérieur (46a) est espacé vers l'intérieur relativement au premier bord s'étendant longitudinalement (117a) du noyau absorbant (103) sur une distance de 5 mm à 10 mm, quand elle est mesurée le long de la ligne centrale s'étendant transversalement (14) de la serviette (10).

9. Serviette hygiénique selon la revendication 1, dans laquelle le bord arqué interne (48b) est espacé vers l'intérieur relativement au bord arqué extérieur (46b) et le bord arqué extérieur (46b) est espacé vers l'intérieur relativement au second bord s'étendant longitudinalement (117b) du noyau absorbant (103).

10. Serviette hygiénique (10) selon la revendication 9, dans laquelle le bord arqué extérieur (46b) est espacé vers l'intérieur relativement au second bord s'étendant longitudinalement (117b) du noyau absorbant (103), sur une distance de 5 mm à 10 mm quand elle est mesurée le long de la ligne centrale s'étendant transversalement (14) de la serviette (10).

11. Serviette hygiénique (10) selon l'une quelconque des revendications 6 à 10, dans laquelle un bord arqué interne (48a) de la première partie (34) et un bord arqué interne (48b) de la seconde partie (36) sont espacés l'un de l'autre sur une distance de 2 cm à 3 cm, comme mesurée le long de la ligne centrale s'étendant transversalement (14).

12. Serviette hygiénique (10) selon l'une quelconque des revendications précédentes, dans laquelle la zone dénuée de gaufrage (54) est située dans une zone de la serviette (10) adaptée pour être disposée sur l'ouverture vaginale pendant l'utilisation.

13. Serviette hygiénique (10) selon l'une quelconque des revendications précédentes, dans laquelle la zone dénuée de gaufrage (54) a une largeur de 2 cm à 3 cm, comme mesurée le long de la ligne centrale s'étendant transversalement (14).

14. Serviette hygiénique (10) selon l'une quelconque des revendications précédentes, dans laquelle la zone dénuée de gaufrage (54) a une longueur de 5 cm à 10 cm, quand elle est mesurée le long de la ligne centrale s'étendant longitudinalement (12).

15. Serviette hygiénique (10) selon l'une quelconque des revendications précédentes, comprenant en outre un premier canal d'extrémité (76) situé dans la première zone d'extrémité (24) et un second canal d'extrémité (78) situé dans la seconde zone d'extrémité (25).

16. Serviette hygiénique (10) selon la revendication 15, dans laquelle le premier et le second canaux d'extrémité (76 et 78) sont espacés l'un de l'autre dans une direction longitudinale de la serviette (10), le premier canal d'extrémité (76) étant structuré et disposé de façon à être sensiblement lié à la première partie (27) du premier motif gaufré (26) et le second canal d'extrémité (78) étant structuré et disposé de façon à être sensiblement lié à la seconde partie (29) du premier motif gaufré (26).

17. Serviette hygiénique (10) selon chacune des revendications 1 à 11, dans laquelle
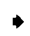 la zone dénuée de gaufrage (54) est située entre le bord arqué interne (48a) de la première partie (34) et le bord arqué interne (48b) de la seconde partie (36), la zone dénuée de gaufrage (54) est située dans une zone de la serviette (10) adaptée pour être disposés sur l'ouverture vaginale pendant l'utilisation, la zone dénuée de gaufrage (54) ayant une largeur de 2 cm à 3 cm, comme mesurée le long de la ligne centrale s'étendant transversale (14) et une longueur de 5 cm à 10 cm quand elle est mesurée le long de la ligne centrale s'étendant longitudinalement (12) ;
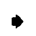 chacune des protubérances (42) du second motif gaufré (32) a un sommet (56) qui est d'1 mm à 2 mm au dessus d'une surface inférieure (38) d'un canal tournant (40) ;
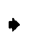 une surface de la serviette (10) dans la zone dénuée de gaufrage (54) est située à une distance de 0,25 mm à 2 mm au dessus de chacun des sommets (56) de chacune des protubérances (42) ;
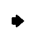 chacune des protubérances (42) du second motif gaufré (32) a une superficie comprise entre 0,49 cm² et 1,44 cm² et chacun des canaux (40) du second motif gaufré (32) a une largeur comprise entre 0,5 mm et 3 mm ;
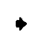 un premier canal d'extrémité (76) situé dans la
première zone d'extrémité (24) et un second canal d'extrémité (78) situé dans la seconde zone d'extrémité (25), le premier et le second canaux d'extrémité (76 et 78) étant espacés l'un de l'autre dans une direction longitudinale de la serviette (10), chacun des premier et second canaux d'extrémité (76 et 78) étant sensiblement en forme de U, le premier canal d'extrémité (76) étant structuré et disposé de façon à être sensiblement lié à la première partie (27) du premier motif gaufré (26) et le second canal d'extrémité (78) étant structuré et disposé de façon à être sensiblement lié à la seconde partie (29) du premier motif gaufré (26), chacun des premier et second canaux (76 et 78) ayant une longueur de 8 cm à 12 cm, comme mesurée le long d'un chemin du canal respectif, une largeur de 2 à 4 mm, et une profondeur de 1 mm à 2 mm, comme mesurée depuis une surface de la serviette (10).
